# FASCICULE DE BREVET EUROPEEN

(11) **EP 0 934 059 B1**
(45) Date de publication et mention de la délivrance du brevet: **16.07.2003**
(21) Numéro de dépôt: 97912248.8
(22) Date de dépôt: 24.10.1997
(51) Int. Cl.: C08F 22/14, C08F 22/20, A61K 9/51

(54) **PROCEDE DE PREPARATION DE NANOPARTICULES DE METHYLIDENE MALONATE, NANOPARTICULES CONTENANT EVENTUELLEMENT UNE OU PLUSIEURS MOLECULES BIOLOGIQUEMENT ACTIVES**
METHYLIDEN-MALONAT NANOPARTIKEL, VERFAHREN ZU DEREN HERSTELLUNG, UND DIESE ENTHALTENDE PHARMAZEUTISCHE PRAEPARATE
METHOD FOR PREPARING MALONATE METHYLIDENE NANOPARTICLES, NANOPARTICLES OPTIONALLY CONTAINING ONE OR SEVERAL BIOLOGICALLY ACTIVE MOLECULES

(30) Priorité: 25.10.1996 FR 9613039
(43) Date de publication de la demande: 11.08.1999
(73) Titulaire: Virsol, 75116 Paris (FR)
(72) Inventeur: BRU-MAGNIEZ, Nicole, F-75016 Paris (FR); GUILLON, Xavier, F-47000 Agen (FR); BRETON, Pascal, F-45510 Tigy (FR); COUVREUR, Patrick, F-91140 Villebon sur Yvette (FR); LESCURE, François, F-92500 Rueil Malmaison (FR); ROQUES-CARMES, Claude, F-25000 Besançon (FR); RIESS, Gérard, F-68200 Mulhouse (FR)
(74) Mandataire: Hubert, Philippe
(86) Numéro de dépôt international: FR9701907
(87) Numéro de publication internationale: WO98018455

(56) Documents cités:
- EP-A- 0 007 895
- WO-A-96/02278

## Description

La présente invention a pour objet un procédé de préparation de nanoparticules formées d'un composé méthylidène malonate polymérisé, lesdites nanoparticules, contenant éventuellement une ou plusieurs molécules biologiquement actives, ainsi que les compositions pharmaceutiques les contenant.

Par "nanoparticules", on entend des particules submicroniques ayant un diamètre inférieur à 500 manomètres. Des nanoparticules formées par polymérisation en émulsion d'un cyanoacrylate d'alkyle sont décrites dans le brevet EP 0 007 895. Le procédé utilisé dans la préparation de ces particules de cyanoacrylate d'alkyle repose sur la polymérisation (anionique) du monomère qui a lieu spontanément et en milieu aqueux. La préparation suivant le même principe (polymérisation anionique en émulsion) de nanoparticules constituées d'un polymère de méthylidène malonate est décrite notamment dans F. Lescure et al, Pharm. Res., 1994, 11, 1270-1276. Ces monomères, dont la préparation est décrite dans le brevet EP 0 283 364, ont une structure proche de celle des cyanoacrylates mais la fonction nitrile de ces derniers est remplacée par un ester ou un ester d'ester. Comme les cyanoacrylates, ils polymérisent à froid en milieu aqueux et peuvent être biodégradables.

Les nanoparticules de méthylidène malonate ainsi obtenues présentent cependant certains désavantages.

En effet, la polymérisation en émulsion des méthylidènes malonates sous forme de nanoparticules aboutit, en phase aqueuse et à pH légèrement acide, à la formation d'oligomères, majoritairement de type trimère ou tétramère, hautement biodégradables. Ces espèces moléculaires sont partiellement hydrosolubles, de sorte que la dispersion de ces nanoparticules en milieu aqueux aboutit à leur solubilisation et à la perte rapide de la structure particulaire (P. Breton et al., Eur. J. Pharm. Biopharm., 1996, 42, 95-103). Lorsqu'une molécule biologiquement active est associée aux nanoparticules de méthylidène malonate, elle est donc susceptible d'être libérée très rapidement après l'administration, suite à l'effet de dilution dans le torrent circulatoire qui entraîne la solubilisation rapide des oligomères formant la matrice particulaire, éventuellement avant d'arriver au site d'action du principe actif.

Certaines expériences ont montré que la polymérisation à pH basique permettait la formation de polymères de masses moléculaires plus élevées tout en conservant la taille des nanoparticules. Cependant, de telles synthèses se caractérisent par :
- l'impossibilité d'obtenir des polymères de Mw< 10 000, et a fortiori de Mw< 8000, constitutifs de nanoparticules individualisées, sans formation d'agrégats et sans la présence importante d'espèces oligomériques,
- l'impossibilité de constituer à pH élevé (pH> 7) des polymères de Mw> 20 000 et a fortiori de Mw supérieur, sans la formation inévitable d'aggrégats rendant l'administration intravasculaire de ces préparations impossible.

On entend par Mw la masse moléculaire moyenne en masse (ou masse moléculaire moyenne) définie ainsi : Mw = Σ ni. Mi² / Σ ni. Mi et par Mp la masse moléculaire de l'espèce quantitativement majoritaire.

Dans la suite de la description, la masse moléculaire est exprimée en équivalents polystyrènes (Ep).

Ce procédé de préparation ne convient donc pas si l'on désire préparer des nanoparticules de polyméthylidène malonate constituées de :
- polymères de masse moléculaire moyenne comprise entre 5000 et 10 000, notamment d'environ 8000,
- polymères de masse moléculaire moyenne supérieure à 20 000 sans formation d'agrégats.

La présente invention consiste donc à préparer des nanoparticules de méthylidène malonate ayant un diamètre inférieur à 500 nm, en particulier de 100 à 500 nm, formées d'espèces moléculaires homogènes de masses situées dans une large gamme (Mw compris entre 2 000 et 80 000). Le principe consiste à solubiliser le monomère dans une phase organique aprotique miscible à l'eau mais qui, dans les conditions de préparation des nanoparticules, forme avec le milieu aqueux de polymérisation un mélange non solvant du polymère formé.

Par "phase organique aprotique" ou "solvant organique aprotique", on entend une phase organique ou un solvant sans proton labile capable d'initier un anion.

Les avantages de ce procédé de préparation suivant l'invention sont nombreux:
- il permet une dispersion plus homogène du monomère dans le milieu de polymérisation,
- il utilise des solvants non chlorés et facilement éliminables car volatils,
- il évite la formation d'aggrégats de polymères,
- il donne lieu à des rendements de polymérisation élevés,
- il permet la constitution de polymères de masse moléculaires homogènes situées dans une large gamme (Mw de 2000 à 100000, notamment de 2000 à 80000) en formant des nanoparticules ayant un diamètre inférieur à 500 nm.

En outre, le procédé permet l'utilisation d'agents de dispersion tels que des agents tensioactifs non ioniques ou des polymères colloïdes protecteurs, ce qui aboutit à l'obtention de particules ayant des propriétés de surface modulables.

Enfin, la masse moléculaire des oligomères/polymères formant les nanoparticules suivant l'invention peut être parfaitement maîtrisée en jouant sur les conditions suivantes de préparation :
- la concentration en monomère dans la phase organique,
- le pH et la molarité du milieu de polymérisation,
- la nature et la concentration de l'agent de dispersion,
- le rapport volumique phase aqueuse (milieu de polymérisation) /phase organique,
- le mode d'introduction du mélange organique dans la phase aqueuse.

L'invention concerne donc dans un ler aspect un procédé de préparation de nanoparticules formées d'un polymère statistique d'au moins un composé de formule (I) 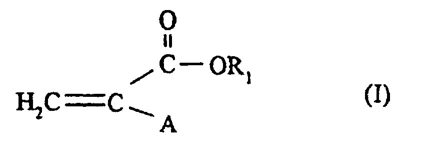 dans laquelle
- A représente
   . un groupe 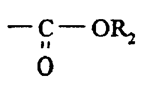 ou
   . un groupe 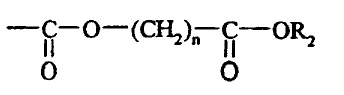
- R₁ et R₂, identiques ou différents, représentent un groupe C₁-C₆ alkyle linéaire ou ramifié ;
   n = 1, 2, 3, 4 ou 5 ;
caractérisé en ce que le ou les monomère(s) est (sont), préalablement à la polymérisation, solubilisé(s) dans un solvant organique aprotique miscible à l'eau formant avec le milieu de polymérisation un mélange non solvant du polymère formé.

Dans un aspect avantageux, l'invention concerne un procédé de préparation de nanoparticules formées d'un polymère d'un composé de formule (I) 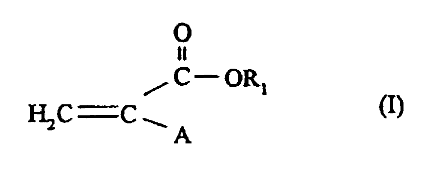 dans laquelle
- A représente
   . un groupe 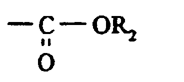 ou
   . un groupe 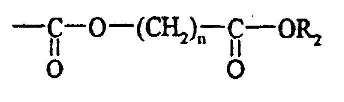
- R₁ et R₂, identiques ou différents, représentent un groupe C₁-C₆ alkyle linéaire ou ramifié ;
   n = 1, 2, 3, 4 ou 5 ;
caractérisé en ce que le monomère est, préalablement à la polymérisation, solubilisé dans un solvant organique aprotique miscible à l'eau formant avec le milieu de polymérisation un mélange non solvant du polymère formé.

Selon un aspect particulier, le procédé selon l'invention permet la préparation de nanoparticules ayant un diamètre inférieur à 500 nm, de préférence compris entre 100 et 500 nm, et une masse moléculaire moyenne (Mw) comprise entre 1000 et 100000, notamment entre 1000 et 80000, en particulier entre 2000 et 80000, de préférence entre 8000 et 80000.

En particulier, le procédé selon l'invention comprend les étapes consistant à:
- préparer une solution d'au moins un composé de formule (I) dans un solvant organique aprotique miscible à l'eau,
- ajouter, sous agitation, cette phase organique à un milieu de polymérisation aqueux à un pH compris entre 4,5 et 10,
- récupérer les nanoparticules ainsi obtenues après homogénéisation du mélange et évaporation sous vide du solvant organique.

On peut également ajouter le milieu de polymérisation aqueux à la phase organique contenant le monomère préalablement solubilisé et selon un autre aspect, le procédé selon l'invention comprend les étapes consistant à :
- préparer une solution d'au moins un composé de formule (I) dans un solvant organique aprotique miscible à l'eau,
- ajouter, sous agitation, à cette phase organique un milieu de polymérisation aqueux à un pH compris entre 4,5 et 10,
- récupérer les nanoparticules ainsi obtenues après homogénéisation du mélange et évaporation sous vide du solvant organique.

Comme illustré plus loin dans les exemples, le pH du milieu de polymérisation est choisi en fonction de la masse moléculaire du polymère que l'on souhaite préparer.

Avantageusement, le mélange de la phase organique et du milieu aqueux est homogénéisé par agitation continue pendant environ 30 min, puis on complète éventuellement la préparation par de l'eau distillée.

Le polymère formé précipite dans le milieu de polymérisation et peut être récupéré par exemple par filtration. On peut ensuite conditionner et lyophiliser la suspension nanoparticulaire ainsi obtenue.

Le solvant organique aprotique utilisé pour disperser le ou les monomère(s) doit être un solvant dudit (desdits) monomère(s) qui soit également miscible à l'eau. Ce solvant est de préférence choisi parmi l'acétone, l'acétonitrile, le dioxanne et le tétrahydrofurane, l'acétone étant particulièrement préférée.

Des aspects préférés du procédé sont les suivants :
- la concentration en monomère(s) de formule (I) dans le solvant organique est de l'ordre de 30 mg/ml à 150 mg/ml ;
- la molarité du milieu de polymérisation est de l'ordre de 1/30 M à 1/3 M;
- le rapport volumique phase aqueuse / phase organique est compris entre 3/1 et 20/1, de préférence entre 3/1 et 15/1.

Avantageusement, le milieu de polymérisation contient un ou plusieurs agents tensioactifs ou colloïdes protecteurs.

Les agents tensioactifs peuvent par exemple être des tensioactifs ioniques ou non ioniques. On utilisera de préférence des agents tensioactifs non ioniques choisis parmi les copolymères de polyoxyéthylène, de polyoxypropylène, les poloxamers et les polysorbates. En tant qu'agents colloïdes protecteurs, on utilisera de préférence les dérivés polysaccharidiques tels que les dextrans, les dérivés cellulosiques hydrosotubles ; les polyéthylène glycols ; l'alcool polyvinylique.

De préférence, le composé polymérisé pour former les nanoparticules selon le procédé de l'invention est un composé de formule (I) dans laquelle : A représente un groupe 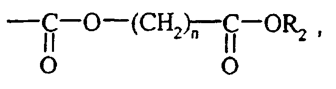 n = 1 et R₁ = R₂ = éthyle.

Dans un autre aspect préféré, le composé polymérisé pour former les nanoparticules selon le procédé de l'invention est un composé de formule (I) dans laquelle : A représente un groupe 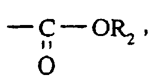 et R₁ = R₂ = propyle.

Avantageusement, on peut également polymériser de manière aléatoire un mélange de composés de formule (I) dans laquelle A est un groupe 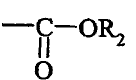 ou un groupe 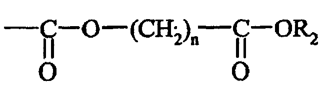 tels que définis plus haut.

Dans un 2ème aspect, l'invention concerne des nanoparticules formées d'un polymère statistique d'au moins un composé de formule (I), dans laquelle A représente un groupe 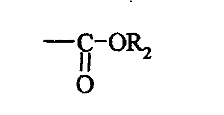 et R₁ = R₂ = propyle, ayant un diamètre inférieur à 500 nm, de préférence compris entre 100 et 500 nm et une masse moléculaire moyenne (Mw) comprise entre 8000 et 100000, de préférence entre 8000 et 80000.

L'invention concerne en particulier des nanoparticules formées d'un polymère d'un composé de formule (I), dans laquelle A représente un groupe 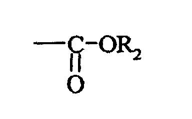 et R₁ = R₂ = propyle, ayant un diamètre inférieur à 500 nm, de préférence compris entre 100 et 500 nm et une masse moléculaire moyenne (Mw) comprise entre 8000 et 80000.

Avantageusement, lesdites nanoparticules peuvent être constituées d'un polymère statistique d'un mélange de composés de formule (I) dans laquelle A est un groupe 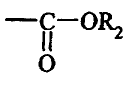 tel que défini plus haut.

Selon un aspect ultérieur de l'invention, lesdites nanoparticules comprennent dans leur réseau polymérique une ou plusieurs molécules biologiquement actives telles que mentionnées plus haut.

En effet, dans un aspect avantageux du procédé selon l'invention, la phase organique (lorsqu'il s'agit d'une molécule biologiquement active insoluble dans l'eau) ou le milieu de polymérisation peut contenir une ou plusieurs molécules biologiquement actives.

Par "molécule biologiquement active", on entend de manière non limitative toute molécule ou macromolécule ayant une activité biologique prophylactique ou curative, in vitro ou in vivo, notamment un agent anti-infectieux, en particulier un agent antiseptique, antibiotique, antiviral, antiparasitaire ou antimitotique, notamment anticancéreux.

Des agents antibiotiques ou antiseptiques utilisables peuvent être par exemple la rifampicine et la colistine.

A titre d'exemples d'agents antiviraux, on peut citer de manière non limitative la didanosine, la ribavirine, la zidovudine, l'acyclovir, le ganciclovir, le foscarnet, la vidarabine et la zalcitabine.

Le cis-plastine, le 5-fluorouracile ou le taxol peuvent par exemple être utilisés en tant qu'agents anticancéreux. Un autre agent antitumoral avantageux est la créatine phosphate dont l'activité est décrite dans la demande EP 0 614 366.

L'invention concerne également les compositions pharmaceutiques contenant lesdites nanoparticules comprenant une ou plusieurs molécules biologiquement actives, en association avec un véhicule pharmaceutiquement acceptable.

Les compositions selon l'invention peuvent être des compositions administrables par exemple par voie orale, sublinguale, sous-cutanée, intramusculaire, intraveineuse, transdermique, locale, rectale, pulmonaire ou nasale.

Les formes d'administration appropriées comprennent notamment les formes par voie orale telles que les comprimés, les gélules, les poudres, les granules et les solutions ou suspensions orales, les formes d'administration sublinguale et buccale, ainsi que les formes d'administration sous-cutanée, intramusculaire, intraveineuse, intranasale ou intraoculaire et les formes d'administration rectale.

L'invention est illustrée par les exemples ci-dessous, dans laquelle la préparation des particules est effectuée à température ambiante (environ 21°C). La taille, ou diamètre, des nanoparticules a été mesuré avec un compteur à diffusion d'un rayon laser (Coulter Electronic Inc., USA). La masse moléculaire des polymères a été déterminée par chromatographie en perméation de gel.

### Exemple 1 :

500 mg de monomère 1-éthoxycarbonyl-1-éthoxycarbonylméthylène-oxycarbonyléthène (Laboratoires UPSA /CARPIBEM, France) préalablement désorbés du SO₂ pendant 3 h sous 25 mbars sont dissous dans 5,55 ml d'acétone. Cette solution est ensuite mélangée progressivement et sous agitation magnétique avec 50 ml d'un milieu aqueux tamponné à pH 8 (Na₂HPO₄/KH₂PO₄, 1/15 M) et contenant 500 mg de dextran 70 (FLUKA CHEMIE, Suisse). La polymérisation quasi-instantanée produit une opacification du mélange qui présente un effet Tyndall caractéristique des solutions colloïdales. L'agitation est maintenue pendant 30 minutes après l'introduction complète de la phase organique. Ensuite, 50 ml d'eau distillée contenant 2,5 g de glucose ou de tréhalose (protecteurs de colloïdes et cryoprotecteurs) sont ajoutés à la suspension nanoparticulaire et le mélange est soumis à une évaporation sous vide afin d'éliminer l'acétone et de réduire le volume de la suspension aqueuse à 50 ml. Après filtration sur filtre papier (diamètre des pores 5 à 15 µm), la préparation est lyophilisée. Mesurées par diffusion d'un rayon laser, les particules contenues dans le filtrat ont un diamètre de 288 nm. La masse moléculaire moyenne (Mw) du méthylidène malonate constituant la matrice polymère des particules est évaluée à 67 000 par chromatographie en perméation de gel.

### Exemple 2 : Etude de la variation du pH

On procède suivant la technique décrite dans l'exemple 1, mais en faisant uniquement varier le pH du tampon phosphate. Les résultats sont rapportés dans le tableau 1 ci-dessous, dans lequel Mp est la masse moléculaire de l'espèce principale et Mw est la masse moléculaire moyenne du polymère.

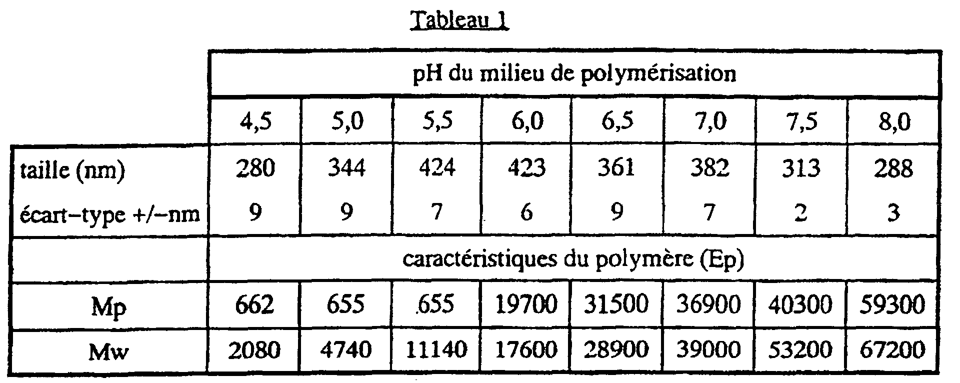

Les résultats montrent que la masse moléculaire moyenne des polymères constituant les nanoparticules augmente régulièrement avec le pH du milieu de polymérisation.

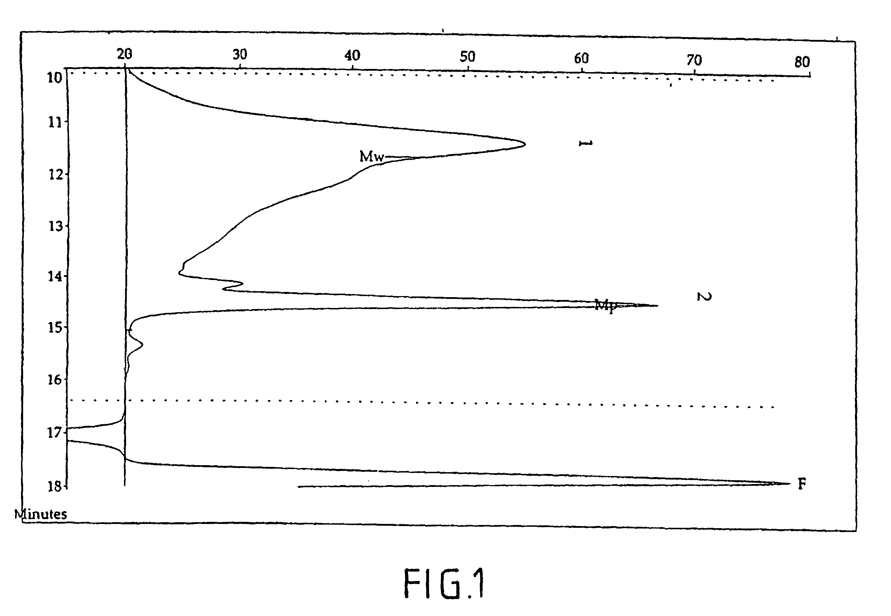

Le profil de chromatographic en perméation de gel de la Figure 1 représente la distribution de masse moléculaire du polymère préparé à pH 5,5 (concentration : 90 mg/ml). On observe en 1 un large pic correspondant aux espèces de haute masse moléculaire moyenne (Mw) et en 2 un pic étroit correspondant aux oligomères minoritaires (trimères et tétramères majoritaires).

Les lignes en pointillés délimitent la portion analysable du chromatogramme. Le pic F est celui du toluène utilisé comme étalon interne et le pic négatif correspond aux traces d'eau.

### Exemple 3 : Etude de la variation de la concentration du monomère.

On procède suivant la technique décrite dans l'exemple 1, mais en faisant uniquement varier la concentration du monomère dans l'acétone. Les résultats sont rapportés dans le tableau 2 ci-dessous :

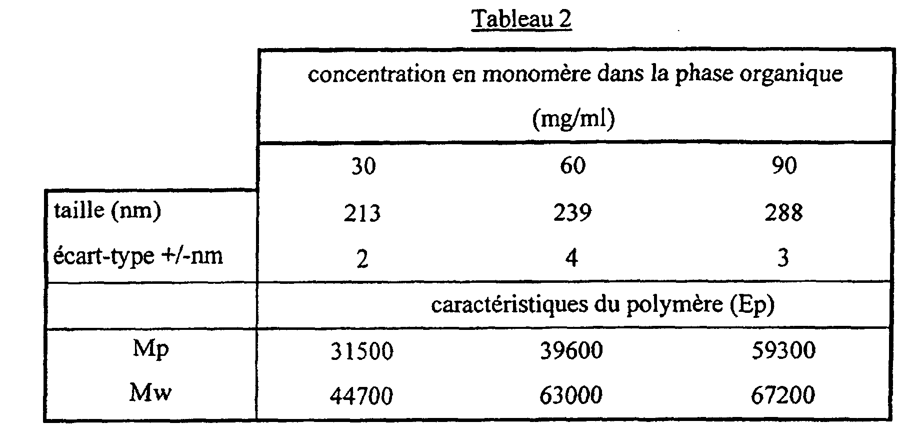

Les résultats montrent que la masse moléculaire de l'espèce principale (Mp), de même que la masse moléculaire moyenne (Mw) des polymères constituant les nanoparticules augmentent régulièrement avec la concentration en monomère dans la phase organique.

### Exemple 4 :

On procède selon les exemples 1 à 3 mais en remplaçant le dextran 70 colloïde protecteur par un tensioactif non ionique, le Pluronic F68 (BASF Corporation, USA).

Les résultats sont rapportés dans le tableau 3 ci-dessous.

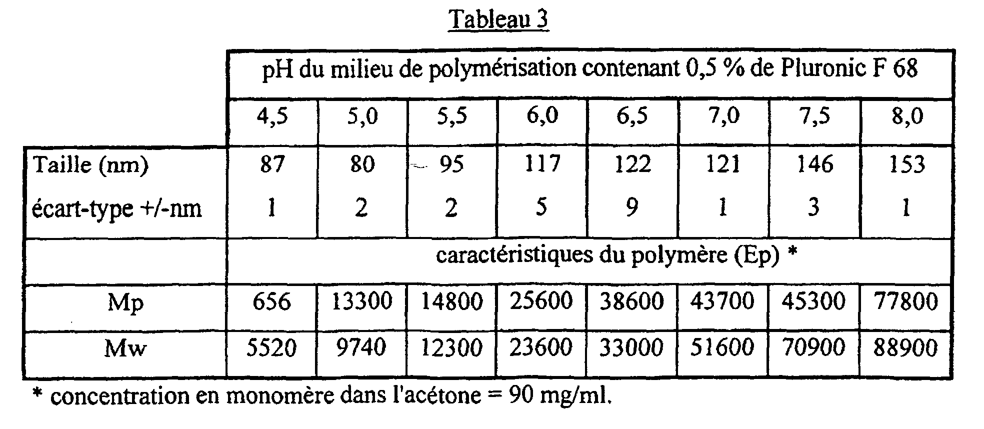

Les résultats montrent, pour des mêmes conditions de pH :
- une augmentation de la masse moléculaire de l'espèce principale (Mp) et de la masse moléculaire moyenne (Mw) des polymères constituant les nanoparticules en présence du tensioactif par rapport au colloïde protecteur,
- une diminution de la taille de ces mêmes nanoparticules en présence du tensioactif par rapport au colloïde protecteur.

### Exemple 5 : Etude de la molarité du milieu de polymérisation

Selon le procédé décrit dans l'exemple 1, on introduit 500 mg de monomère dissous dans 16,6 ml d'acétone, dans un tampon phosphate (Na₂HPO₄/KH₂PO₄) de molarité croissante, et contenant par ailleurs 0,5 % de Pluronic F68.

Les résultats sont rapportés dans le tableau 4 ci-dessous :

**Tableau 4**

| molarité | taille des nanoparticules (nm) | écart-type (nm) | Mp (Ep) | Mw (Ep) |
|---|---|---|---|---|
| 0,033 M | 127 | 2 | 15200 | 12500 |
| 0,066 M | 123 | 1 | 14600 | 12400 |
| 0,133 M | 124 | 1 | 653 | 9790 |
| 0,267 M | 179 | 3 | 660 | 8690 |

Les résultats montrent une diminution de la masse moléculaire moyenne (Mw) des polymères constituant les nanoparticules proportionnellement à une augmentation de la molarité du milieu.

### Exemple 6 :

Des nanoparticules sont préparées suivant les exemples 1 à 3 et comparées à des nanoparticules préparées selon le procédé décrit par Lescure et al., Pharm. Res. 1994, 11, 1270 - 1276. Pour cela, on introduit 100 mg de monomère sous agitation dans 10 ml d'un milieu tampon phosphate (Na₂HPO₄/KH₂PO₄, 1/15 M) de pH 5 à 8. Les résultats sont rapportés dans le tableau 5 ci-dessous dans lequel les oligomères sont définis comme toute espèce moléculaire de masse moléculaire inférieure ou égale à 920.

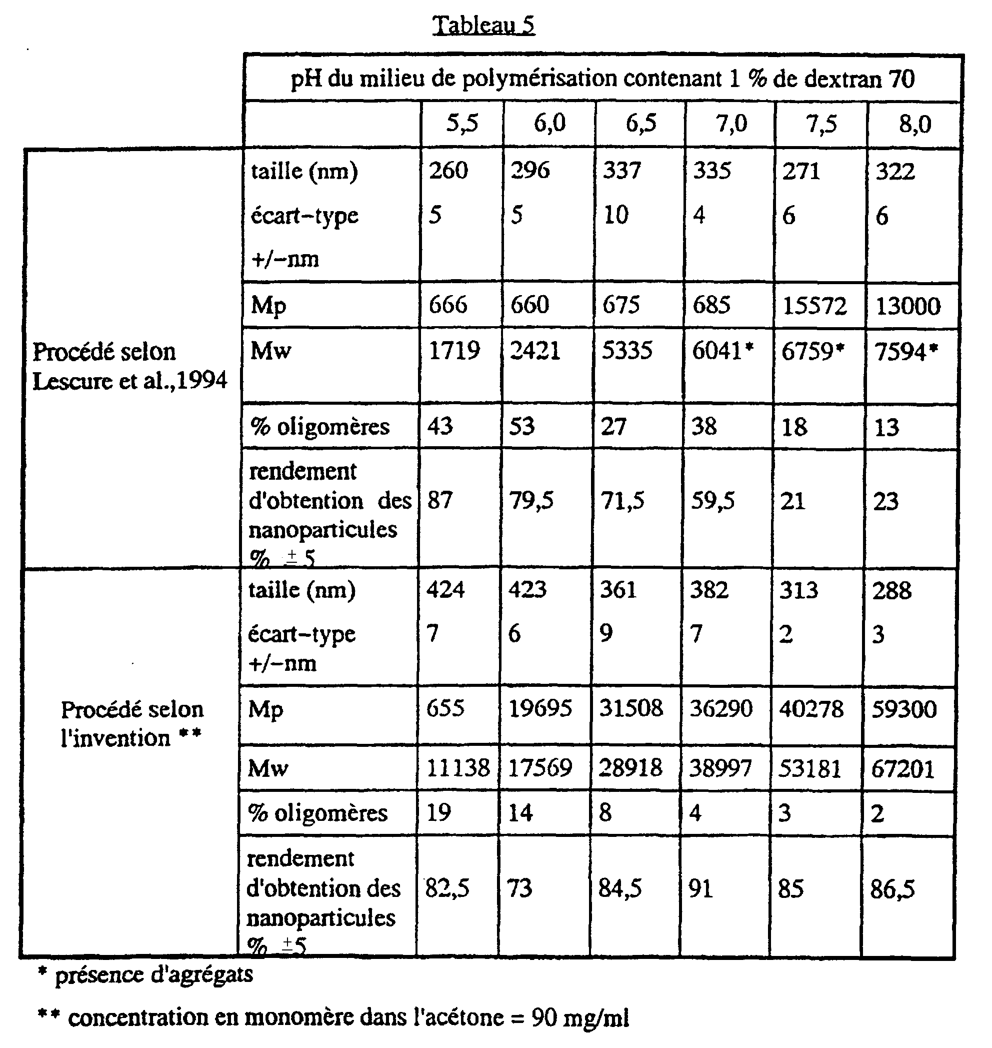

Les résultats montrent que, pour toute condition expérimentale de pH identique :
- la masse moléculaire moyenne (Mw) des polymères constituant les nanoparticules fabriquées selon Lescure et al. est inférieure à celle du polymère obtenu selon le procédé de l'invention;
- les taux d'oligomères (trimères-tétramères) constitutifs des polymères sont significativement inférieurs pour les nanoparticules préparées selon le procédé de l'invention;
- les rendements de polymérisation sous forme de nanoparticules sont supérieurs pour le procédé de l'invention par rapport au procédé selon Lescure et al (la formation d'agrégats se traduit par de faibles rendements à pH basique pour le procédé selon Lescure et al).

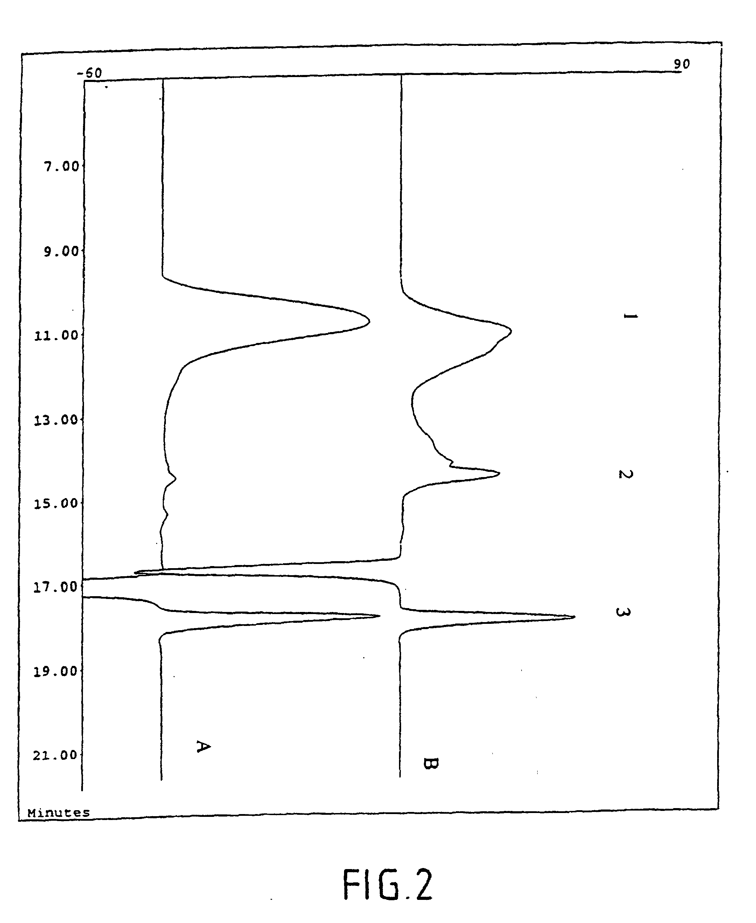

Le profil de chromatographie en perméation de gel de la Figure 2 représente les distributions de masse moléculaire des polymères préparés à pH 7,5 selon le procédé de l'invention d'une part (tracé A), et selon le procédé de Lescure et al d'autre part (tracé B). Mis à part le pic 3 correspondant au toluène, on observe pour le tracé A un pic 1 unique correspondant à l'espèce principale (Mp=40278) alors que pour le tracé B, on observe également la présence d'un pic 2 significatif correspondant aux oligomères (trimères et tétramères).

### Exemple 7 :

50 ml d'un milieu aqueux tamponné à pH 6,5 (Na₂HPO₄/KH₂PO₄ 1/15 M) et contenant 0,5 % de Pluronic F68 (BASF Corporation, USA) sont ajoutés progressivement et sous agitation magnétique à 5,55 ml d'une solution de 500 mg de monomère 1-éthoxycarbonyl-1-éthoxycarbonyl méthylèneoxycarbonyléthène (LABORATOIRES UPSA/CARPIBEM, France) préalablement désorbés du SO₂ pendant 3 h sous 25 mbars dans 5,55 ml d'acétone. L'agitation est maintenue pendant 16 h après l'introduction complète de la phase organique. Ensuite, 50 ml d'eau distillée contenant 2,5 g de glucose ou de tréhalose (colloïdes protecteurs et cryoprotecteurs) sont ajoutés à la suspension nanoparticulaire et le mélange est soumis à une évaporation sous vide afin d'éliminer l'acétone et de réduire le volume de la suspension aqueuse à 50 ml. Après filtration sur filtre en papier (diamètre des pores 5 à 15 µm), la préparation est lyophilisée. Le diamètre des particules contenues dans le filtrat est mesuré par diffusion d'un rayon laser. La masse moléculaire moyenne (Mw) du méthylidène malonate constituant la matrice polymère des particules est évaluée par chromatographie en perméation de gel.

Les résultats sont rapportés dans le tableau 6 ci-dessous, dans lequel Mp est la masse moléculaire de l'espèce principale et Mw est la masse moléculaire moyenne du polymère.

Le rendement est déterminé par le rapport entre la quantité de monomère introduit dans le milieu réactionnel et la quantité de polymère constituant les nanoparticules.

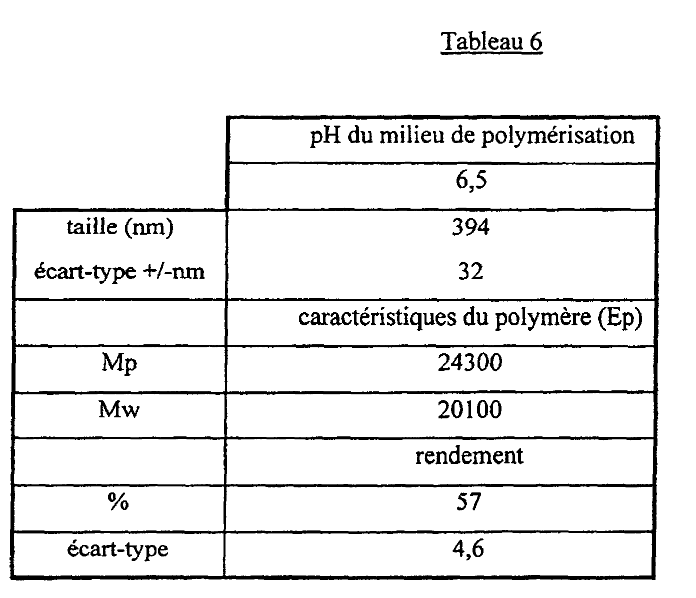

### Exemple 8 : Utilisation de différents solvants.

On procède suivant le procédé de l'exemple 1, mais en utilisant l'acétone, l'acétonitrile ou le tétrahydrofuranne (THF) comme solvant du monomère.

Les résultats sont rapportés dans le tableau 7 ci-dessous.

**Tableau 7**

| Solvant | Granulométrie moyenne (nm) | rendement (%) | Mw |
|---|---|---|---|
| Acétone | 253 | 74 | 54 100 |
| Acétonitrile | 197 | 69 | 31 700 |
| THF | 191 | 70 | 30 300 |

### Exemple 9 : Etude du rapport volumique eau / solvant

On procède suivant le procédé de l'exemple 1, mais en faisant varier le rapport volumique eau / acétone.

Les résultats sont rapportés dans le tableau 8 ci-dessous :

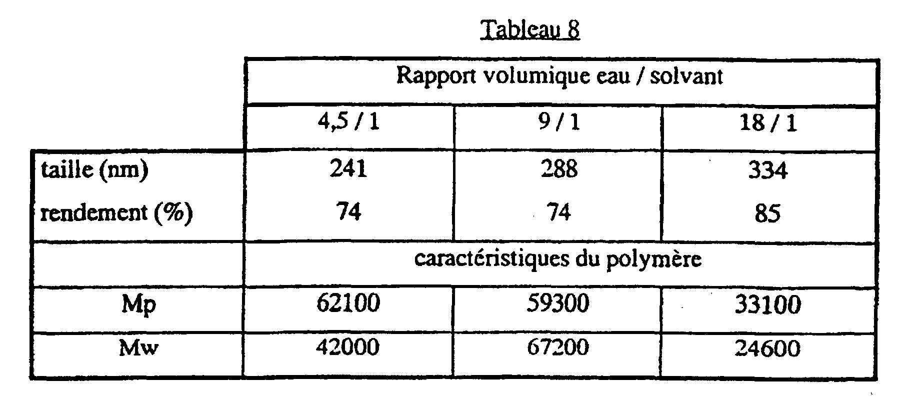

### Exemple 10 : Mise en oeuvre du procédé à pH 10.

Les essais ont été réalisés dans un milieu aqueux à pH = 10 en présence soit d'agent tensio-actif soit de colloïde protecteur et ce, soit selon le procédé de l'exemple 1, soit selon le procédé de l'exemple 7.

### 1) essai 1

100 mg de monomère 1-éthoxycarbonyl-1-éthoxycarbonylméthylène-oxycarbonyléthène sont dissous dans 1 ml d'acétone.

Cette solution est ensuite ajoutée progressivement, et sous agitation magnétique, dans 10 ml d'un milieu aqueux à pH = 10 et contenant 100 mg de Dextran 70.

La polymérisation est instantanée. L'agitation est maintenue pendant 30 minutes après introduction de la totalité de la phase organique. Ensuite, 10 ml d'eau distillée sont additionnés à la suspension de nanoparticules, et le mélange est soumis à une évaporation sous vide afin d'éliminer l'acétone. Puis le milieu est centrifugé (v = 10 000 tr/min, 10 min à 4°C).

### 2) essai 2

Le protocole expérimental est identique à celui de l'essai 1 mais en remplaçant le Dextran 70 par du Pluronic F68.

### 3) essai 3

10 ml d'un milieu aqueux à pH = 10 contenant 100 mg de Dextran 70 sont ajoutés progressivement et sous agitation magnétique dans une phase organique constituée de 100 mg de monomère et de 1 ml d'acétone. La polymérisation est instantanée. L'agitation est maintenue pendant 30 minutes après introduction de la totalité de la phase aqueuse. Ensuite, 10 ml d'eau distillée sont additionnés à la suspension nanoparticulaire et le mélange est soumis à une évaporation sous vide afin d'éliminer l'acétone. Puis le milieu est centrifugé ( v = 10 000 tr/min, 10 min à 4°C).

### 4) essai 4

Le protocole expérimental est identique à celui de l'essai 3 mais en remplaçant le Dextran 70 par du Pluronic F68. Après centrifugation, les nanoparticules contenues dans le culot sont analysées par chromatographie d'exclusion stérique pour déterminer leur masse moléculaire moyenne en poids (Mw).

Les résultats sont rapportés dans le tableau 9 ci-après :

**Tableau 9**

| | Mw | Granulométrie (nm) |
|---|---|---|
| Essai 1 | 8 800 | 240 |
| Essai 2 | 6 900 | 245 |
| Essai 3 | 1 400 | 316 |
| Essai 4 | 1 850 | 333 |

### Exemple 11 :

On procède suivant la technique de polymérisation décrite dans l'exemple 1, mais en utilisant du 1,1-propoxycarbonyléthène (Laboratoires UPSA/CARPIBEM, France) ci-après dénommé MM 33, seul ou en mélange avec du monomère 1-éthoxycarbonyl-1-éthoxycarbonylméthylèneoxy-carbonyléthène (Laboratoires UPSA / CARPIBEM, France), ci-après dénommé MM 2.1.2. Les résultats sont rapportés dans le tableau 10 ci-dessous, dans lequel Mp est la masse moléculaire de l'espèce principale et Mw est la masse moléculaire moyenne du polymère.

**Tableau 10**

| | Rapport MM 3.3/MM 2.12 | | | |
|---|---|---|---|---|
| | 100/0 | 75/25 | 50/50 | 25/75 |
| Taille | 123 | 223 | 298 | 155 |
| Rendement (%) | 77 | 73 | 80 | 78 |

| | Caractéristiques du polymère | | | |
|---|---|---|---|---|
| Mp | 44764 | 92090 | 37467 | 21727 |
| Mw | 44122 | 89793 | 37467 | 21727 |

### Exemple 12 : Préparation de nanoparticules contenant de la rifampicine

5 mg de rifampicine base (Sigma) sont dissous dans 1 ml d'acétone auquel on ajoute 90 mg de monomère 1-éthoxycarbonyl-1-éthoxycarbonylméthylèneoxycarbonyléthène (LABORATOIRES UPSA /CARPIBEM, France) préalablement désorbé du SO₂ pendant 3 h sous 25 mbars. A l'aide d'une pipette en verre, cette solution est ensuite ajoutée progressivement et sous agitation constante (750 rpm) à 9 ml de milieu aqueux tamponné à pH 6,0 à l'aide d'un tampon phosphate (Na₂HPO₄/KH₂PO₄ 0,066M) et contenant 90 mg de dextran 70 (1% p/v). Après 18 h de polymérisation à 20°C, 9 ml d'eau distillée contenant 5% de D-glucose sont additionnés sous agitation à la suspension nanoparticulaire, puis le mélange est soumis à une évaporation sous vide à l'aide d'un Rotavapor (20°C, 25 mbars) afin d'éliminer l'acétone et de réduire le volume de la suspension aqueuse à 9 ml. La préparation est ensuite lyophilisée; la congélation a lieu à -30°C et la sublimation à +20°C pendant 36 h à une pression de 0,05 mbar.

La taille des nanoparticules et la concentration en rifampicine sont mesurés avant et après lyophilisation. La taille est mesurée par diffusion d'un rayon laser. Le dosage de la rifampicine est réalisé par chromatographie liquide haute performance couplée à un spectrophotomètre. La phase mobile est composée d'un mélange méthanol/acétate d'ammonium 0,05M (65:35), le pH est ajusté à 7,3, le débit est fixé à 1ml/min et l'absorption est lue à 254 nm. Le taux de rifampicine non liée aux nanoparticules est mesuré dans le surnageant obtenu après ultracentrifugation de la suspension nanoparticulaire (80000g, 1 h à 4°C). La quantité de rifampicine fixée aux nanoparticules correspond à la fraction présente dans le culot, qui est dissous dans le THF avant de procéder au dosage direct de la rifampicine.

On obtient les résultats suivants :
- taille des nanoparticules contenant de la rifampicine : 266±63 nm avant lyophilisation et 282±54 nm après lyophilisation;
- pourcentage de fixation de la rifampicine : 8,5±0,5% avant et après lyophilisation.

### Exemple 13 : Préparation de nanoparticules contenant de la colistine

On procède de la même manière que dans l'exemple 12, mais le principe actif étant hydrosoluble, celui-ci est incorporé dans le milieu de polymérisation à une concentration de 0,5 mg/ml avant addition de la phase organique.

La taille des nanoparticules contenant de la colistine mesurée par diffusion d'un rayon laser est de 282±65 nm après évaporation et de 283±26 nm après conservation à +4°C pendant 4 jours. Dosée selon la technique de diffusion sur gélose (S.P. Gotoff et al., Antimicrob. Agents Chemother, 1962, 107-113), la colistine est retrouvée à la concentration de 15 µg/ml dans le surnageant obtenu après ultracentrifugation de la suspension nanoparticulaire (80000g, 1 h à 4°C) : la fraction non liée aux nanoparticules est donc évaluée à 3% de la quantité totale de colistine ajoutée.

### Exemple 14 :

Préparation de nanoparticules contenant de l'azido-thymidine (AZT) (Sigma Aldrich Chimie, France).

240 mg de monomère 1-éthoxycarbonyl-1-éthoxycarbonylméthylèneoxycarbonyléthène (Laboratoires UPSA / CARPIBEM, France) préalablement désorbé du SO₂ pendant 3 heures sous 25 mbars, sont dissous dans 2,5 ml d'acétone. A l'aide d'une propipette, cette solution est ensuite ajoutée progressivement et sous agitation constante à 22,5 ml de milieu aqueux tamponné à pH 8,0 à l'aide d'un tampon phosphate (Na₂HPO₄/KH₂PO₄ 0,066M) et contenant 225 mg de dextran 70 (1 % p/v), ainsi que le principe actif hydrosoluble à une concentration de 0,53 mg/ml. Après 18 heures de polymérisation à 20°C, 22,5 ml d'eau déminéralisée contenant 5 % de D-glucose sont additionnés sous agitation à la suspension nanoparticulaire, puis le mélange est soumis à une évaporation sous vide à l'aide d'un Rotavapor (20°C, 25 mbars) afin d'éliminer l'acétone et de réduire le volume de la suspension aqueuse à 39,0 ml. La préparation est ensuite lyophilisée ; la congélation a lieu à -30°C et la sublimation à +20°C pendant 36 heures à une pression de 0,05 mbar.

La taille des nanoparticules contenant de l'AZT mesurée par diffusion d'un rayon laser est de 255 ± 63 nm avant lyophilisation. Le taux d'AZT dans le surnageant après centrifugation de la suspension nanoparticulaire (12 000 tr/min, 1 h à 4°C) est dosé par spectrophotométrie UV à 266 nm. On obtient une concentration de 98 µg/ml: la fraction non liée aux nanoparticules est donc évaluée à 31,9 % de la quantité totale d'AZT ajouté. La fraction d'AZT liée aux nanoparticules est donc de 68,1 %.

### Exemple 15 : Préparation de nanoparticules contenant de la créatine phosphate (Boehringer Mannheim).

L'encapsulation de la créatine phosphate est réalisée selon la technique de l'exemple 14. La taille des nanoparticules contenant de la créatine phosphate mesurée par diffusion d'un rayon laser est de 275 ± 260 nm avant lyophilisation. Le dosage de la créatine phosphate est réalisé par chromatographie liquide haute performance couplée à un spectrophotomètre. La phase mobile est composée d'un tampon phosphate (KH₂PO₄, 0,05M) ajusté à pH 3,3. Le débit est fixé à 2 ml/min et l'absorption est lue à 200 nm.

Le taux de créatine phosphate non liée aux nanoparticules est mesuré dans le surnageant obtenu après centrifugation de la suspension nanoparticulaire (12 000 tr/min, 1 h à 4°C). La créatine phosphate est retrouvée à une concentration de 463 µg/ml dans le surnageant: la fraction non liée aux nanoparticules est donc évaluée à 81 % de la quantité totale de créatine phosphate ajoutée. La fraction de créatine phosphate liée aux nanoparticules est donc de 19 %.

### Exemple 16 : Préparation de nanoparticules contenant du 5-fluorouracile (5-FU)

L'encapsulation du 5-FU (Sigma Aldrich Chimie, France) est réalisée selon la technique de l'exemple 14. La taille des nanoparticules contenant le 5-FU mesurée par diffusion d'un rayon laser est de 516 ± 88 nm avant lyophilisation. Dosé par spectrophotométrie UV à 266 nm, le 5-FU est retrouvée à une concentration de 70 µg/ml dans le surnageant obtenu après centrifugation de la suspension nanoparticulaire (12 000 tr/min, 1 h à 4°C) : la fraction non liée aux nanoparticules est donc évaluée à 23,3 % de la quantité totale de 5-FU ajouté. La fraction de 5-FU liée aux nanoparticules est donc de 76,7 %.

## Revendications

1. Procédé de préparation de nanoparticules formées d'un polymère statistique d'au moins un composé de formule (I) 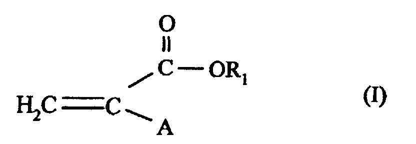 dans laquelle
- A représente
. un groupe 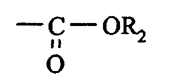 ou
. un groupe 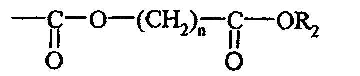
- R₁ et R₂, identiques ou différents, représentent un groupe C₁-C₆ alkyle linéaire ou ramifié;
- n = 1, 2, 3, 4 ou 5 ;
**caractérisé en ce que** le ou les monomère(s) est (sont), préalablement à la polymérisation, solubilisé(s) dans un solvant organique aprotique miscible à l'eau formant avec le milieu de polymérisation un mélange non solvant du polymère formé.

2. Procédé selon la revendication 1 de préparation de nanoparticules formées d'un polymère d'un composé de formule (I) 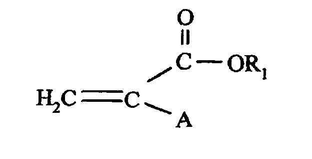 dans laquelle
- A représente
. un groupe 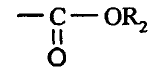 ou
. un groupe 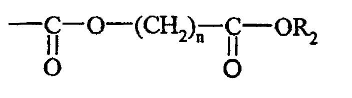
- R₁ et R₂, identiques ou différents, représentent un groupe C₁-C₆ alkyle linéaire ou ramifié ;
- n = 1, 2, 3, 4 ou 5,
**caractérisé en ce que** le monomère est, préalablement à la polymérisation, solubilisé dans un solvant organique aprotique miscible à l'eau formant avec le milieu de polymérisation un mélange non solvant du polymère formé.

3. Procédé selon l'une des revendications 1 ou 2, pour la préparation de nanoparticules ayant un diamètre inférieur à 500 nm, de préférence compris entre 100 et 500 nm, et une masse moléculaire moyenne (Mw) comprise entre 1000 et 100000, notamment entre 1000 et 80000, en particulier entre 2000 et 80000, de préférence entre 8000 et 80000.

4. Procédé selon l'une quelconque des revendications 1 à 3, **caractérisé en ce qu'**il comprend les étapes consistant à :
- préparer une solution d'au moins un composé de formule (I) dans un solvant organique aprotique miscible à l'eau,
- ajouter, sous agitation, cette phase organique à un milieu de polymérisation aqueux à un pH compris entre 4,5 et 10,
- récupérer les nanoparticules ainsi obtenues après homogénéisation du mélange et évaporation sous vide du solvant organique.

5. Procédé selon l'une quelconque des revendications 1 à 3, **caractérisé en ce qu'**il comprend les étapes consistant à :
- préparer une solution d'au moins un composé de formule (I) dans un solvant organique aprotique miscible à l'eau,
- ajouter, sous agitation, à cette phase organique un milieu de polymérisation aqueux à un pH compris entre 4,5 et 10,
- récupérer les nanoparticules ainsi obtenues après homogénéisation du mélange et évaporation sous vide du solvant organique.

6. Procédé selon l'une quelconque des revendications 1 à 5, **caractérisé en ce que** le solvant organique aprotique est choisi parmi l'acétone, l'acétonitrile, le dioxanne et le tétrahydrofurane.

7. Procédé selon l'une quelconque des revendications 1 à 6, **caractérisé en ce que** la concentration en composé(s) de formule (I) dans le solvant organique est de l'ordre de 30 mg/ml à 150 mg/ml.

8. Procédé selon l'une quelconque des revendications 1 à 7, **caractérisé en ce que** la molarité du milieu de polymérisation est de l'ordre de 1/30 M à 1/3 M.

9. Procédé selon l'une quelconque des revendications 1 à 8, **caractérisé en ce que** le milieu de polymérisation contient un ou plusieurs agents tensioactifs ou colloïdes protecteurs.

10. Procédé selon la revendication 9, **caractérisé en ce que** les agents tensioactifs sont des tensioactifs non ioniques choisis parmi les copolymères de polyoxyéthylène, de polyoxypropylène, les poloxamers et les polysorbates.

11. Procédé selon la revendication 9 ou 10, **caractérisé en ce que** les agents colloïdes protecteurs sont choisis parmi les dextrans, les dérivés cellulosiques hydrosolubles, les polyéthylène glycols et l'alcool polyvinylique.

12. Procédé selon l'une quelconque des revendications 1 à 11, **caractérisé en ce que** la phase organique ou le milieu de polymérisation contient une ou plusieurs molécules biologiquement actives.

13. Procédé selon l'une quelconque des revendications 1 à 12, **caractérisé en ce que** le composé polymérisé est un composé de formule (I) dans laquelle A représente un groupe 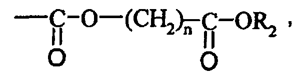 R₁ = R₂ = éthyle et n = 1.

14. Procédé selon l'une quelconque des revendications 1 à 12, **caractérisé en ce que** le composé polymérisé est un composé de formule (I) dans laquelle A représente un groupe 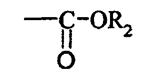 et R₁ = R₂ = propyle.

15. Procédé selon l'une quelconque des revendications 1 à 12, **caractérisé en ce qu'**on polymérise de manière aléatoire un mélange de composés de formule (I) dans laquelle A est un groupe 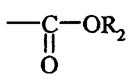 ou un groupe 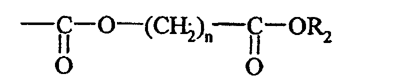 tels que définis dans la revendication 1.

16. Nanoparticules formées d'un polymère statistique d'au moins un composé de formule (I) 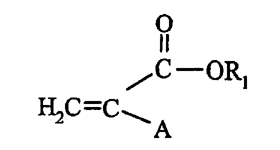 dans laquelle
- A représente un groupe 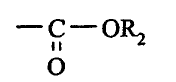 et R₁ = R₂ = propyle, ayant un diamètre inférieur à 500 nm, de préférence compris entre 100 et 500 nm et une masse moléculaire moyenne (Mw) comprise entre 8 000 et 100000, de préférence entre 8000 et 80000.

17. Nanoparticules formées d'un polymère d'un composé de formule (I) 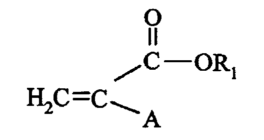 dans laquelle
- A représente un groupe 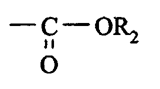 et R₁ = R₂ = propyle, ayant un diamètre inférieur à 500 nm, de préférence compris entre 100 et 500 nm et une masse moléculaire moyenne (Mw) comprise entre 8000 et 80000.

18. Nanoparticules selon l'une quelconque des revendications 16 ou 17, **caractérisées en ce qu'**elles comprennent une ou plusieurs molécules biologiquement actives.

19. Composition pharmaceutique contenant à titre de principe actif des nanoparticules selon la revendication 18 en association avec un véhicule pharmaceutiquement acceptable.

## Patentansprüche

1. Verfahren zur Herstellung von Nanopartikeln, die aus einem statistischen Polymer aus mindestens einer Verbindung der Formel (I) gebildet werden 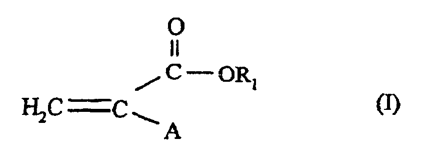 wobei
A einen Rest 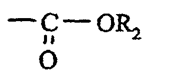 oder einen Rest 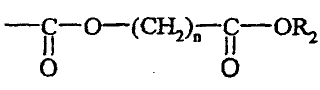 darstellt,
R₁ und R₂, die gleich oder unterschiedlich sein können, einen linearen oder verzweigten C₁-C₆-Alkylrest darstellen;
n gleich 1, 2, 3, 4 oder 5 ist;
**dadurch gekennzeichnet, dass** das oder die Monomer(e) vor der Polymerisation in einem mit Wasser mischbaren, aprotischen organischen Lösungsmittel gelöst wird bzw. werden, das mit dem Polymerisationsmedium ein Gemisch bildet, in dem das gebildete Polymer unlöslich ist.

2. Verfahren gemäß Anspruch 1 zur Herstellung von Nanopartikeln, die aus einem Polymer einer Verbindung der Formel (I) gebildet werden 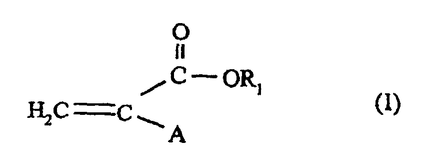 wobei
A einen Rest 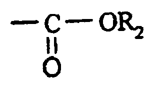 oder einen Rest 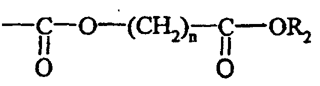 darstellt,
R₁ und R₂, die gleich oder unterschiedlich sein können, einen linearen oder verzweigten C₁-C₆-Alkylrest darstellen;
n gleich 1, 2, 3, 4 oder 5 ist;
**dadurch gekennzeichnet, dass** das Monomer vor der Polymerisation in einem mit Wasser mischbaren aprotischen organischen Lösungsmittel gelöst wird, das mit dem Polymerisationsmedium ein Gemisch bildet, in dem das gebildete Polymer unlöslich ist.

3. Verfahren gemäß einem der Ansprüche 1 oder 2 zur Herstellung von Nanopartikeln, die einen Durchmesser von weniger als 500 nm, vorzugsweise zwischen 100 und 500 nm, und ein mittleres Molekulargewicht (Mw) zwischen 1000 und 100000, vornehmlich zwischen 1000 und 80000, insbesondere zwischen 2000 und 80000 und vorzugsweise zwischen 8000 und 80000 aufweisen.

4. Verfahren gemäß einem der Ansprüche 1 bis 3, **dadurch gekennzeichnet, dass** es die folgenden Schritte umfasst:
- Herstellen einer Lösung mindestens einer Verbindung der Formel (I) in einem mit Wasser mischbaren, aprotischen organischen Lösungsmittel,
- Zugeben dieser organischen Phase unter Rühren zu einem wässrigen Polymerisationsmedium mit einem pH-Wert zwischen 4,5 und 10 und
- Gewinnen der Nanopartikel, die so nach Homogenisieren des Gemischs und Entfernen des organischen Lösungsmittels im Vakuum erhalten werden.

5. Verfahren gemäß einem der Ansprüche 1 bis 3, **dadurch gekennzeichnet, dass** es die folgenden Schritte umfasst:
- Herstellen einer Lösung mindestens einer Verbindung der Formel (I) in einem mit Wasser mischbaren, aprotischen organischen Lösungsmittel,
- Zugeben eines wässrigen Polymerisationsmediums mit einem pH-Wert zwischen 4,5 und 10 unter Rühren zu dieser organischen Phase und
- Gewinnen der Nanopartikel, die so nach Homogenisieren des Gemischs und Entfernen des organischen Lösungsmittels im Vakuum erhalten werden.

6. Verfahren gemäß einem der Ansprüche 1 bis 5, **dadurch gekennzeichnet, dass** das aprotische organische Lösungsmittel aus Aceton, Acetonitril, Dioxan und Tetrahydrofuran ausgewählt ist.

7. Verfahren gemäß einem der Ansprüche 1 bis 6, **dadurch gekennzeichnet, dass** die Konzentration an Verbindung(en) der Formel (I) in dem organischen Lösungsmittel in der Größenordnung von 30 mg/ml bis 150 mg /ml liegt.

8. Verfahren gemäß einem der Ansprüche 1 bis 7, **dadurch gekennzeichnet, dass** die Molarität des Polymerisationsmediums in der Größenordnung von 1/30 M bis 1/3 M liegt.

9. Verfahren gemäß einem der Ansprüche 1 bis 8, **dadurch gekennzeichnet, dass** das Polymerisationsmedium eines oder mehrere oberflächenaktive Mittel oder Schutzkolloide enthält.

10. Verfahren gemäß Anspruch 9, **dadurch gekennzeichnet, dass** die oberflächenaktiven Mittel nicht-ionische Tenside, ausgewählt aus Polyoxyethylen- und Polyoxypropylen-Copolymeren, Poloxameren und Polysorbaten, sind.

11. Verfahren gemäß Anspruch 9 oder 10, **dadurch gekennzeichnet, dass** die Schutzkolloide ausgewählt sind aus Dextranen, wasserlöslichen Cellulosederivaten, Polyethylenglycolen und Polyvinylalkohol.

12. Verfahren gemäß einem der Ansprüche 1 bis 11, **dadurch gekennzeichnet, dass** die organische Phase oder das Polymerisationsmedium eines oder mehrere biologisch aktive Moleküle enthält.

13. Verfahren gemäß einem der Ansprüche 1 bis 12, **dadurch gekennzeichnet, dass** die polymerisierte Verbindung eine Verbindung der Formel (I) ist, wobei A einen Rest 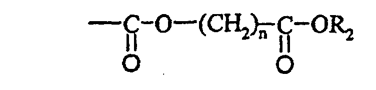 darstellt, R₁ und R₂ jeweils ein Ethylrest sind und n gleich 1 ist.

14. Verfahren gemäß einem der Ansprüche 1 bis 12, **dadurch gekennzeichnet, dass** die polymerisierte Verbindung eine Verbindung der Formel (I) ist, wobei A einen Rest 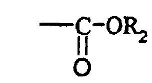 darstellt und R₁ und R₂ jeweils ein Propylrest sind.

15. Verfahren gemäß einem der Ansprüche 1 bis 12, **dadurch gekennzeichnet, dass** ein Gemisch von Verbindungen der Formel (I) in statistischer Sequenz polymerisiert wird, wobei A einer der Reste 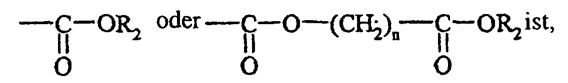 so wie in Anspruch 1 definiert.

16. Nanopartikel, die aus einem statistischen Polymer mindestens einer Verbindung der Formel (I) gebildet sind 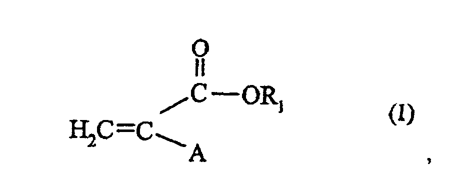 wobei
A einen Rest 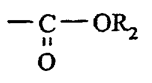 darstellt und R₁ und R₂ jeweils ein Propylrest sind. und die einen Durchmesser von weniger als 500 nm, vorzugsweise zwischen 100 und 500 nm, und ein mittleres Molekulargewicht (Mw) zwischen 8000 und 100000, vorzugsweise zwischen 8000 und 80000 aufweisen.

17. Nanopartikel, die aus einem Polymer einer Verbindung der Formel (I) gebildet sind, 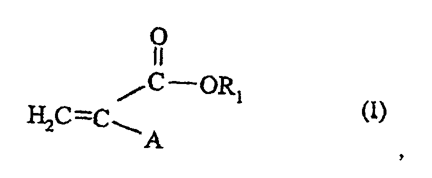 wobei
A einen Rest 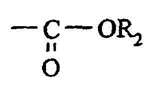 darstellt und R₁ und R₂ jeweils ein Propylrest sind, und die einen Durchmesser von weniger als 500 nm, vorzugsweise zwischen 100 und 500 nm, und ein mittleres Molekulargewicht (Mw) zwischen 8000 und 80000 aufweisen.

18. Nanopartikel gemäß einem der Ansprüche 16 oder 17, **dadurch gekennzeichnet, dass** sie ein oder mehrere biologisch aktive Moleküle umfassen.

19. Arzneimittel, das als Wirkstoff Nanopartikel gemäß Anspruch 18 in Verbindung mit einer pharmazeutisch verträglichen Trägersubstanz enthält.

## Claims

1. A method for the preparation of nanoparticles formed from a random polymer of at least one compound of formula (I) 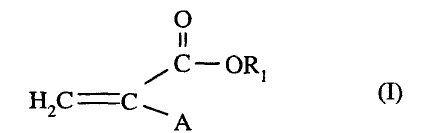 in which
- A represents a 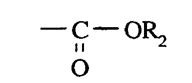 group or a 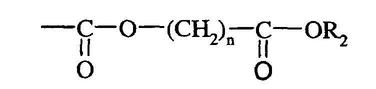 group;
- R₁ and R₂, identical or different, represent a linear or branched C₁-C₆ alkyl group; n = 1, 2, 3, 4 or 5 ;
**characterised in that** the monomer(s) is (are), before the polymerisation, dissolved in a water-miscible aprotic organic solvent forming, with the polymerisation medium, a non-solvent mixture of the polymer formed.

2. The method according to claim 1 for the preparation of nanoparticles formed from a polymer of a compound of formula (I) 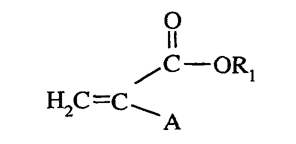 in which:
- A represents a 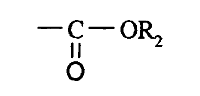 group or a 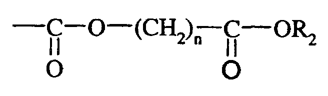 group;
- R₁ and R₂, identical or different, represents a linear or branched C₁-C₆ alkyl group;
- n = 1, 2, 3, 4 or 5,
**characterised in that** before the polymerisation, the monomer is dissolved in a water-miscible aprotic organic solvent forming, with the polymerisation medium, a non-solvent mixture of the polymer formed.

3. The method according to one of claims 1 or 2, for the preparation of nanoparticles having a diameter of less than 500 nm, preferably between 100 and 500 nm, and an average molecular mass (Mw) between 1000 and 100000, notably between 1000 and 80000, in particular between 2000 and 80000, preferably between 8000 and 80000.

4. The method according to any one of claims 1 to 3, **characterised in that** it comprises the steps consisting in :
- preparing a solution of at least one compound of formula (I) in a water-miscible aprotic organic solvent,
- adding, with stirring, this organic phase to an aqueous polymerisation medium at a pH between 4.5 and 10,
- recovering the nanoparticles thus obtained after homogenisation of the mixture and evaporating the organic solvent *in vacuo*.

5. The method according to any one of claims 1 to 3, **characterised in that** it comprises the steps consisting in :
- preparing a solution of at least one compound of formula (I) in a water-miscible aprotic organic solvent,
- adding, with stirring, to this organic phase, an aqueous polymerisation medium at a pH between 4.5 and 10,
- recovering the nanoparticles thus obtained after homogenisation of the mixture and evaporating the organic solvent *in vacuo*.

6. The method according to any one of claims 1 to 5, **characterised in that** the aprotic organic solvent is selected from acetone, acetonitrile, dioxane and tetrahydrofuran.

7. The method according to any one of claims 1 to 6, **characterised in that** the concentration of compound(s) of formula (I) in the organic solvent is of the order of 30 mg/ml to 150 mg/ml.

8. The method according to any one of claims 1 to 7, **characterised in that** the molarity of the polymerisation medium is of the order of 1/30 M to 1/3 M.

9. The method according to any one of claims 1 to 8, **characterised in that** the polymerisation medium contains one or more surfactants or colloid protectors.

10. The method according to claim 9, **characterised in that** the surfactants are non-ionic surfactants selected from copolymers of polyoxyethylene, of polyoxypropylene, poloxamers and polysorbates.

11. The method according to claim 9 or 10, **characterised in that** the colloid protector agents are selected from dextrans, hydrosoluble cellulose derivatives, polyethylene glycols and poly(vinyl alcohol).

12. The method according to any one of claims 1 to 11, **characterised in that** the organic phase or the polymerisation medium contains one or more biologically active molecules.

13. The method according to any one of claims 1 to 12, **characterised in that** the polymerised compound is a compound of formula (I) in which A represents a 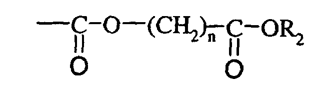 group, R₁ = R₂ = ethyl and n = 1.

14. The method according to any one of claims 1 to 12, **characterised in that** the polymerised compound is a compound of formula (I) in which A represents a 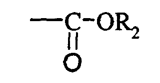 group and R₁ = R₂ = propyl.

15. The method according to any one of claims 1 to 12, **characterised in that** a mixture of compounds of formula (I) in which A is a 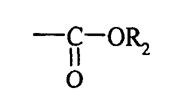 group or a 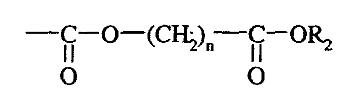 group as defined in claim 1, is random polymerised.

16. Nanoparticles formed from a random polymer of at least one methylidene malonate compound of formula (I) 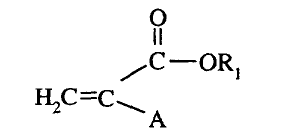 in which
- A represents a 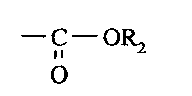 group, and R₁ = R₂ = propyl,
having a diameter of less than 500 nm, preferably between 100 and 500 nm and an average molecular mass (Mw) between 8000 and 100000, preferably between 8000 and 80000.

17. Nanoparticles formed from a polymer of a methylidene malonate compound of formula (I) 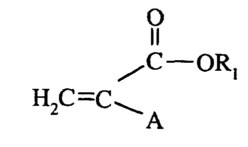 in which
- A represents a 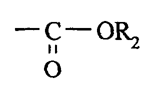 group, and R₁ = R₂ = propyl,
having a diameter of less than 500 nm, preferably between 100 and 500 nm and an average molecular mass (Mw) between 8000 and 80000.

18. The nanoparticles according to any one of claims 16 or 17, **characterised in that** they comprise one or more biologically active molecules.

19. Pharmaceutical composition containing nanoparticles according to claim 18 as active principle in association with a pharmaceutically acceptable vehicle.
